# EUROPEAN PATENT APPLICATION

(11) **EP 2 002 730 A1**
(43) Date of publication of application: **17.12.2008**
(21) Application number: 07252354.1
(22) Date of filing: 11.06.2007
(51) Int. Cl.: A23L 1/00, A61K 31/202, A61P 1/16

(54) **Pinolenic acid compositions for treating fatty liver**

(71) Applicant: Lipid Nutrition B.V., 1521 AZ Wormerveer (NL)
(72) Inventor: Einerhand, Alaxandra W.C., 1521 AZ Wormerveer (NL); Gambelli, Luisa, 1521 AZ Wormerveer (NL); Schmid, Ulrike, 1521 AZ Wormerveer (NL); Zara, Vincenzo, 73100 Lecce (IT)
(74) Representative: Stevens, Ian Edward

(57) **Abstract**

A composition comprising pinolenic acid or a derivative thereof may be used in treating or preventing a condition of the liver. Preferably, the pinolenic acid or derivative represents at least 75 % by weight of the total Δ5-polyunsaturated C18-C20 fatty acids in the composition.

## Description

This invention relates to compositions that may be used for treating and/or preventing a condition of the liver. The compositions contain pinolenic acid or a derivative thereof.

The liver is the largest internal organ in the human body and its functions are essential to the body. A large blood vessel carries nutrient-rich blood from the small intestine directly to the liver. Hepatic cells make up about 60 percent of liver tissue. These specialised liver cells carry out more chemical processes than any other group of cells in the body. They convert sugars and store and release them as needed, thereby regulating blood sugar level, breaking down fats, producing cholesterol, removing ammonia from the body and producing blood proteins, including blood clotting factors. Other functions of hepatic cells are to detoxify drugs and alcohol and to produce bile, which breaks down fats in food.

Fatty liver is a condition of the liver that involves the build-up of fat in liver cells. Fatty liver may be associated with, or may lead to, inflammation of the liver. This can cause scarring and hardening of the liver. When scarring becomes extensive, it is called cirrhosis, and this is a serious condition. It is still not known what causes fat to build up in the liver and a biopsy is typically required to diagnose fatty liver.

Many compounds have been proposed for treating fatty liver. For example, WO 2007/016390 relates to a method for treating a fatty liver disease or disorder in a patient in need thereof. The method comprises administering at least one matrix metalloproteinase ("MMP") inhibitor, such as Marimastat, to the patient.

There remains a need for compounds and compositions that can treat or prevent conditions of the liver, especially compounds and compositions that are generally regarded as safe and are relatively inexpensive to produce.

Pinolenic acid (i.e., 5, 9, 12 C18:3 fatty acid, a fatty acid with 18 carbon atoms having three cis double bonds in the positions 5, 9 and 12) is present in, for example, pine nut oil and fractions thereof (see J Am Oil Chem Soc 1998, 75, p.45-50).

FR-A-2756465 discloses the use of a concentrate with 15% pinolenic acid in food additives. The presence of pinolenic acid is described as providing a hypolipemic effect to the composition and the examples concentrate on reducing serum cholesterol and triglycerides. The weight of the liver is determined in the examples but the document concludes that there is no significant difference in the weight of the liver compared to the control. The composition used in the document was from *Pinus pinaster* and contained a relatively low amount of pinolenic acid compared to other C18-20 Δ5-polyunsaturated fatty acids.

EP-A-1685834 discloses the use of pinolenic acid or a derivative thereof in the manufacture of a composition for weight management by reducing the feeling of hunger and/or increasing satiety.

According to JP-A-61238729, pine nut oil can be used as an anticholesteric agent. Other documents wherein health effects of pinolenic acid are disclosed include JP-A-61058536, wherein a very generic activity beneficial for human health is disclosed. This health activity is not disclosed in further detail. Sugano, Brit J. of Nutr 72 (1994) 775-783, discloses health effects of diets containing pinolenic acid, including hypocholesterolaemic effects, effects on ADP-induced platelet aggregation, on aortic prostacylic production and on blood pressure. EP-A-1088552 describes the use of pinolenic acid as an anti-inflammatory agent.

CN-A-1377673 relates to the use of a pine nut oil for treating cardiac and cerebral vascular diseases and adiposity caused by hyperlipemia as well as diabetes caused by hyperglycemia.

Although many different uses of pinolenic acid are described in the above documents, there is no disclosure that pinolenic acid may be used to treat or prevent conditions of the liver.

It has now surprisingly been found that pinolenic acid and its derivatives can have a beneficial effect on the liver.

Accordingly, the present invention provides the use of pinolenic acid or a derivative thereof in the manufacture of a composition for treating or preventing a condition of the liver.

In another aspect, the invention provides a composition comprising pinolenic acid or a derivative thereof for use in treating or preventing a condition of the liver.

A further aspect of the invention is a method of treating or preventing a liver condition, which comprises administering to a mammal an effective amount of pinolenic acid or a derivative thereof.

The condition of the liver may be the general health of the liver, including for example a reduction in the weight of the liver, or may be fatty liver. Fatty liver disease or disorders include, for example, nonalcoholic fatty liver disease (NAFLD), nonalcoholic steatohepatitis (NASH), alcoholic liver disease (ALD), fatty liver associated with chronic hepatitis infection, total parenteral nutrition-associated liver disease (TPN), steroid treatment, tamoxifen treatment, gastrointestional operations, diabetes and Reye's Syndrome.

Typically, the pinolenic acid or derivative is in the form of a food supplement or a food product.

In a preferred embodiment of the invention, the pinolenic acid or derivative (which term is intended to cover both pinolenic acid and derivatives when both are present) is in a form selected from the free acid, salts, mono-, di- or triglycerides, or mixtures thereof.

Sources of pinolenic acid and its derivatives are available and will be known to those skilled in the art. Preferably, the pinolenic acid or derivative is in the form of pine nut oil or is derived from pine nut oil. The pine nut oil is preferably from *Pinus koraiensis* or from the Siberian pine, *Pinus sibirica.*

The pinolenic acid or derivative may form part of a composition that comprises one or more other components. The pinolenic acid may be used as the single component of the composition, or the major fatty acid component i.e., at least 75 %, more preferably at least 85 %, such as at least 95 % by weight of the fatty acids or their derivatives (calculated as free acid) in the composition are pinolenic acid or one or more derivatives of pinolenic acid. Additionally or alternatively, the pinolenic acid or derivative is in the form of a composition which further comprises one or more additional fatty acids or derivatives thereof selected from the group consisting of linoleic acid, oleic acid, conjugated linoleic acid, enriched isomer mixtures of conjugated linoleic acid, EPA and DHA, and mixtures thereof. The one or more additional fatty acids or derivatives thereof are preferably present as a free fatty acid, salt, mono-, di- or triglyceride or a mixture thereof and more preferably are in the same form (either free acid or derivative) as the pinolenic acid e.g., if the pinolenic acid is present as a glyceride, then the one or more additional fatty acids are also present as glycerides.

In a preferred embodiment of the invention, the pinolenic acid or derivative is in the form of a composition which comprises from 1 to 50 %, more preferably from 5 to 40 %, such as from 10 to 35 %, or from 15 to 30 %, by weight pinolenic acid or derivative thereof, based on the total weight of fatty acids in the composition (calculated as free fatty acid).

Particularly preferred compositions of the invention are those in which the pinolenic acid or derivative is in the form of a composition which additionally comprises from 30 to 70 % by weight linoleic acid or derivative thereof, based on the total weight of fatty acids in the composition (calculated as free fatty acid). Additionally or alternatively, the pinolenic acid or derivative is in the form of a composition which additionally comprises from 10 to 40 % by weight oleic acid or derivative thereof, based on the total weight of fatty acids in the composition (calculated as free fatty acid). Additionally or alternatively, the pinolenic acid or derivative is in the form of a composition which additionally comprises from 1 to 15 % by weight palmitic acid or derivative thereof, based on the total weight of fatty acids in the composition (calculated as free fatty acid). Additionally or alternatively, the composition may comprise from 0.5 to 5 wt% of taxoleic acid or a derivative thereof.

Specific examples of compositions comprising pinolenic acid or a derivative thereof that are useful in the invention include the following:
- compositions comprising from 10 to 35 %, more preferably from 15 to 30 %, by weight pinolenic acid or a derivative thereof, together with from 30 to 70 % by weight linoleic acid or a derivative thereof;
- compositions comprising from 10 to 35 %, more preferably from 15 to 30 %, by weight pinolenic acid or a derivative thereof, together with from 10 to 40 % by weight oleic acid or a derivative thereof;
- compositions comprising from 10 to 35 %, more preferably from 15 to 30 %, by weight pinolenic acid or a derivative thereof, together with from 1 to 15 % by weight palmitic acid or a derivative thereof;
- compositions comprising from 10 to 35 %, more preferably from 15 to 30 %, by weight pinolenic acid or a derivative thereof, together with from 0.5 to 5 wt% of taxoleic acid or a derivative thereof;
- compositions comprising from 10 to 35 %, more preferably from 15 to 30 %, by weight pinolenic acid or a derivative thereof, together with from 30 to 70 % by weight linoleic acid or a derivative thereof and from 10 to 40 % by weight oleic acid or a derivative thereof;
- compositions comprising from 10 to 35 %, more preferably from 15 to 30 %, by weight pinolenic acid or a derivative thereof together with from 30 to 70 % by weight linoleic acid or a derivative thereof and from 1 to 15 % by weight palmitic acid or a derivative thereof;
- compositions comprising from 10 to 35 %, more preferably from 15 to 30 %, by weight pinolenic acid or a derivative thereof together with from 30 to 70 % by weight linoleic acid or a derivative thereof and from 0.5 to 5 wt% of taxoleic acid or a derivative thereof;
- compositions comprising from 10 to 35 %, more preferably from 15 to 30 %, by weight pinolenic acid or a derivative thereof together with from 30 to 70 % by weight linoleic acid or a derivative thereof, from 1 to 15 % by weight palmitic acid or a derivative thereof and from 10 to 40 % by weight oleic acid or a derivative thereof; and
- compositions comprising from 10 to 35 %, more preferably from 15 to 30 %, by weight pinolenic acid or a derivative thereof, together with from 30 to 70 % by weight linoleic acid or a derivative thereof, from 1 to 15 % by weight palmitic acid or a derivative thereof, from 10 to 40 % by weight oleic acid or a derivative thereof and from 0.5 to 5 wt% of taxoleic acid or a derivative thereof.

In these compositions, the amounts of the acids or derivatives are determined as free acid based on the total fatty acid and/or derivative content of the composition. Preferably, the fatty acids are present as free acids (i.e., more than 90 %, preferably more than 95 %, by weight of the fatty acids are present as free fatty acids) or the fatty acids are present as glycerides (more preferably triglycerides) (i.e., more than 90 %, preferably more than 95 %, by weight of the fatty acids are present as glycerides, more preferably triglycerides).

The pinolenic acid or derivative preferably represents at least 65 % by weight, more preferably at least 75 % by weight, even more preferably at least 80 % by weight, of the total Δ⁵-polyunsaturated C18-C20 fatty acids in the composition (calculated as free fatty acid).

The pinolenic acid or derivative may be in the form of a food product. Examples of food products include those comprising a glyceride selected from: liquid oils, including soybean oil, sunflower oil, rape seed oil and cotton seed oil; cocoa butter and cocoa butter equivalents; palm oil and fractions thereof; enzymically made fats; fish oils and fractions thereof; conjugated linoleic acid and enriched isomer mixtures thereof; gamma linoleic acid and enriched mixtures thereof; hardened liquid oils; and mixtures thereof.

Typical food products are selected from the group consisting of: margarines; low fat spreads; very low fat spreads; bicontinuous spreads; water continuous spreads; confectionary products, such as chocolates, coatings or fillings; ice creams; ice cream coatings; ice cream inclusions; dressings; mayonnaises; sauces; bakery fats; shortenings or cheese; meal replacement products; health bars; muesli bars; drinks; dairy products; low carbohydrate products; low calorie products; soups; cereals and milk shakes.

The pinolenic acid or derivative thereof may be used in other product forms. For example, a preferred product form is a food supplement (which term includes nutritional products) which is a soft gel or a hard capsule comprising an encapsulating material. The encapsulating material is typically food grade and is preferably selected from the group consisting of gelatin, glycerol, starch, modified starch, and starch derivatives such as glucose, sucrose, lactose and fructose. The encapsulating material may optionally contain cross-linking or polymerizing agents, stabilizers, antioxidants, light absorbing agents for protecting light-sensitive fills, preservatives and the like. Preferably, the unit dosage of pinolenic acid in the food supplements is from 1 mg to 1000 mg (more preferably from 100 mg to 750 mg). The amount of pine nut oil that is used in a unit dosage form is preferably from 100 mg to 2000 mg, for example 250 mg to 1500 mg (e.g., 750 mg), for example for taking from one to four times a day.

The pinolenic acid used in the present invention may be in the form of a free fatty acid, a derivative of pinolenic acid or mixtures thereof, including mixtures of different derivatives. Derivatives are non-toxic and edible. Derivatives of pinolenic acid, which can be used in the present invention, include salts of pinolenic acid and alkyl esters. Isomers of pinolenic acid and their derivatives can be used in the invention such as, for example, geometric isomers (having one or more trans double bonds; the double bonds in pinolenic acid are all cis). Although the pinolenic acid (and its derivatives) is preferably the 5, 9, 12 all cis isomer, possible derivatives of pinolenic acid also include compounds having 18 carbon atoms and three double bonds with one or more of the three double bonds at a different position in the alkyl chain compared to pinolenic acid, including, for example, gamma linolenic acid, alpha linolenic acid, punicic acid, eleostearic acid, and their salts and alkyl esters. Suitable salts include salts with food grade cations such as sodium salts and calcium salts. Suitable esters include alkyl esters having from one to six carbon atoms. Preferred esters are mono-, di- and tri- glycerides and mixtures thereof.

The compositions comprising pinolenic acid or a derivative thereof that are useful in the invention may be in any suitable form such as a food supplement, a pharmaceutical composition or a food composition. The term composition means that the pinolenic acid or derivative thereof may be present with one or more other components (e.g., as are present in pine nut oil). The one or more other components may be present in admixture with the pinolenic acid or derivative or they may form part of the packaging of the product (e.g., the capsule in which the pinolenic acid or derivative is encapsulated).

A suitable source for the pinolenic acid used in the present invention is pine nut oil or concentrates thereof. For example, glycerides of pinolenic acid can be obtained from pine nut oil or concentrates thereof. Preferably, an oil or concentrate with a content of pinolenic acid or a derivative thereof of more than 15 % by weight or more than 28 % by weight is used (such as up to 50 % by weight).

Pine nut oil can be used in the compositions comprising pinolenic acid or a derivative thereof that are useful in the invention. However, as pine nut oil can contain up to about 26 % by weight of pinolenic acid, it is generally advantageous (in particular for use in food supplements and to enable dosage forms of a smaller size) if concentrates could be obtained with higher levels of pinolenic acid. Concentrates of pinolenic acid or a derivative thereof to be used in the present invention can be prepared by any suitable process. A suitable process is described in EP-A-1088552.

In one suitable process, an enzymic hydrolysis or glycerolysis is performed using an enzyme that can discriminate between fatty acids with a delta 5 double bond and other fatty acids. This process comprises:
i) reacting a glyceride material containing at least 2 % by weight of fatty acid with cis5 double bond with water or glycerol in the presence of an enzyme capable of discriminating between fatty acids containing a delta 5 double bond and other fatty acids;
ii) splitting the reaction mixture into a partial glyceride rich component and a fatty acid rich component;
iii) optionally converting the partial glycerides of step ii) to free fatty acids in the presence of a suitable enzyme;
iv) optionally converting the fatty acid rich component of step ii) to triglycerides by reaction with glycerol in the presence of a suitable catalyst such as a suitable enzyme; and
v) optionally splitting the partial glyceride rich material of step ii) into components that are a) rich in monoglycerides, b) rich in diglycerides and c) rich in triglycerides and then optionally converting the partial glycerides a) and b) into triglycerides by reaction with fatty acids in the presence of a suitable enzyme.

It is preferred to use a glyceride material with a pinolenic acid content of 5 to 50 wt %, preferably 10 to 35 wt % in step i). Examples of such materials are pinolenic oils and concentrates thereof. This process produces a concentrate that contains at least 28 % by weight pinolenic acid.

Enzymes suitable for use in steps i), iii), iv) and v) are lipases. Suitable commercial lipases include *Candida rugosa* lipase; Lipase QL; Lipase SL, Lipase OF; *Rhizopus delemar* lipase; *Rhizopus oryzae* lipase; *Geotrichum candidum* B lipase; and *Rhizomucor miehei* lipase. Preferred enzymes for step i) are *Candida rugosa* lipase and *Geotrichum candidum* B lipase.

Suitable lipases also include Lipozyme IM (a commercial enzyme). The preferred enzyme for use in step iv) is Lipozyme M (from *Rhizomucor miehei).*

The compositions comprising pinolenic acid or a derivative thereof that are useful in the invention may comprise one or more other fatty acids. The term fatty acid, as used herein, refers to straight chain carboxylic acids having from 12 to 24 carbon atoms and being saturated or unsaturated e.g., having 0, 1, 2 or 3 double bonds.

Examples of other fatty acids suitable for use in the present invention include linoleic acid, oleic acid, taxoleic, juniperonic, sciadonic, saturated fatty acids, conjugated linoleic acid (optionally as an enriched isomer mixture) and EPA (eicosapentaenoic) and DHA (docosahexaenoic) (optionally as an enriched isomer mixture of EPA or DHA). Enrichment involves the alteration of the isomer mixture normally present (for example in a natural product), such as an alteration in the relative amounts of different geometrical isomers. In these compositions, the other fatty acid or each of the other fatty acids can independently be present as a free fatty acid or as a derivative thereof (including a mono-, di- or triglyceride and salts), or as a mixture thereof.

The pinolenic acid or derivative thereof is optionally blended with these additional fatty acids or glycerides before being used as a composition in the present invention. When the compositions contain one or more fatty acids and/or glycerides in addition to the pinolenic acid or derivative thereof, the additional fatty acid(s) and/or glycerides are preferably selected from liquid oils, such as soybean oil, sunflower oil, rape seed oil and cotton seed oil; cocoa butter and cocoa butter equivalents; palm oil and fractions thereof; enzymically made fats; fish oils and fractions thereof; conjugated linoleic acid and enriched isomer mixtures; gamma linolenic acid and enriched mixtures thereof; hardened liquid oils; and mixtures thereof.

When producing a food product, the pinolenic acid or derivative thereof can first be blended with structuring components for use in food products. However, this is not essential. These blends can, for example, be applied beneficially in food products as healthy fat compositions.

The addition of pinolenic acid or a derivative thereof or blends containing at least one of the compounds to food products can have a positive effect on the texture, hardness, appearance, rheology, oral melt, flavour impact, spreadability, microstructure (crystal size, droplet size), aeration properties or ease of processing of these food products. The use of a glyceride of pinolenic acid is particularly advantageous in this respect.

Blends containing one or more additional fatty acids or glycerides preferably display solid fat contents measured by NMR-pulse on non stabilised fats of:
- N20 = 1-80, preferably 5-45; and/or
- N35 less than 20, preferably less than 10, most preferably less than 5.

These N values were obtained by melting the fat at 80°C, cooling to 0°C and holding the fat at 0°C for 30 minutes, whereupon the fat was heated to the measurement temperature N and held at that temperature for 30 minutes before measuring the N value.

Blends of fatty acids that are used to produce the compositions of the invention preferably comprise from 1.5 to 60 wt%, more preferably from 28 to 60 wt% of pinolenic acid, from 10 to 60 wt% of linoleic acid and from 5 to 52 wt% of oleic acid, for example 25 to 85 wt% (linoleic plus oleic acid), from 0 to 70 wt%, for example 25 to 65 wt% (trans plus saturated fatty acid). The trans fatty acid content is preferably less than 10 wt%. An example of a suitable blend is one in which the trans plus saturated fatty acid content is less than 10 wt%.

One aspect of the invention is a method of treating or preventing a liver condition, which comprises administering to a mammal an effective amount of pinolenic acid or a derivative thereof. The mammal is preferably in need of said treatment and/or prevention. The invention also provides a composition comprising pinolenic acid or a derivative thereof for use in treating or preventing a condition of the liver. The invention preferably involves a lowering of one or more of liver cholesterol, liver triglycerides and liver phospholipids. The invention may involve a reduction in the weight of the liver. For example, the liver weight may be decreased by at least 20 %. In all cases, the lowering is compared to the liver prior to treatment according to the invention.

The invention is applicable to mammals, preferably humans. Other mammals that may benefit from the compositions of the invention include pets (for example, dogs, cats, horses, rabbits, hamsters and guinea pigs) and farm animals (for example, cattle, sheep and pigs). Dogs and cats are particularly preferred. The method of the invention may be therapeutic.

The pinolenic acid or derivative (typically in the form of a composition e.g., in the form of a foodstuff or food supplement) is preferably consumed once or twice daily over a period of from 5 to 100 days or for longer. The pinolenic acid or derivative preferably represents from 1 to 20 %, more preferably from 5 to 15 %, by weight of the total fatty acids in the diet.

Other examples of compositions useful in the invention are pharmaceutical compositions, such as in the form of tablets, pills, capsules, caplets, multiparticulates including: granules, beads, pellets and micro-encapsulated particles; powders, elixirs, syrups, suspensions and solutions. Pharmaceutical compositions will comprise a pharmaceutically acceptable diluent or carrier. Pharmaceutical compositions are preferably adapted for administration parenterally (e.g., orally). Orally administrable compositions may be in solid or liquid form and may take the form of tablets, powders, suspensions and syrups. Optionally, the compositions comprise one or more flavouring and/or colouring agents. Pharmaceutically acceptable carriers suitable for use in such compositions are well known in the art of pharmacy. Pharmaceutical compositions may contain from 0.1 to 99 % by weight of pinolenic acid or derivative thereof. The compositions are generally prepared in unit dosage form. Preferably, the unit dosage of pinolenic acid is from 1mg to 1000 mg (more preferably from 100 mg to 750 mg). The excipients used in the preparation of these compositions are the excipients known in the art.

The compositions comprising pinolenic acid or a derivative thereof that are useful in the invention may contain other additives that are well known in the art of food and pharmaceutical products including, but not limited to, flavouring ingredients, colouring agents, sweeteners and emulsifiers.

The listing or discussion of an apparently prior-published document in this specification should not necessarily be taken as an acknowledgement that the document is part of the state of the art or is common general knowledge.

The following non-limiting examples illustrate the invention and do not limit its scope in any way. In the examples and throughout this specification, all percentages, parts and ratios are by weight unless indicated otherwise.

### Examples

### Example 1

Male ICR mice were obtained from Harlan at 5 weeks of age and housed individually in animal cages at a temperature of 22 + 1 °C. Animals were fed ad libitum either with a standard diet supplemented with 7.5% corn oil (control) or with the same diet containing 7.5% of a treated pine nut oil composition containing pinolenic acid.

The oil composition is available as PinnoThin^{™} from Lipid Nutrition BV and comprises as triglycerides (in weight %):
Pinolenic acid 16
Linoleic acid 46
Oleic acid 25
Palmitic acid 4
Taxoleic acid 2
Others balance to 100

Table 1 reports the composition of both diets that were prepared each week and stored frozen until use. Body weight, liver weight and food intake were recorded throughout the study, ranging from 2 to 8 weeks of dietary treatment. Experiments were carried out in accordance with local and national guidelines regarding animal experiments.

**Table 1: Composition of diet (in %)**

| | **Corn oil (Control)** | **Pine oil** |
|---|---|---|
| **Proteins** | 17.39 | 17.39 |
| **Lipids** | 12.77 | 12.77 |
| **Fatty acids** | | |
| **16:0** | 1.55 | 1.02 |
| **18:0** | 0.39 | 0.28 |
| **9-18:1** | 3.50 | 2.96 |
| **9.12-18:2** | 6.63 | 6.52 |
| **9.12.15-18:3** | 0.35 | 0.27 |
| **59.12-18:3** | | 1.12 |
| **Other fatty acids** | 0.25 | 0.55 |
| **Carbohydrates** | 52.96 | 52.96 |
| **Sugars** | 4.54 | 4.54 |
| **Mineral + Vitamin mix** | 3.08 | 3.08 |
| **Crude fiber** | 3.51 | 3.51 |
| **Ash** | 5.58 | 5.58 |
| **Kcal/100 g** | 415 | 415 |

The lipids in livers from control and pine oil fed mice were analysed and the results are given in Table 2.

**Table 2: Lipid analysis of livers from control and pine oil fed mice.**

| Weeks | **Triglycerides (mg/g liver)** | | **Cholesterol (mg/g liver)** | | **Phospholipids (mg/g liver)** | |
|---|---|---|---|---|---|---|
| | **Control** | **Pine oil** | **Control** | **Pine oil** | **Control** | **Pine oil** |
| **0** | 3.69±0.38 | - | 1.93±0.23 | - | 9.25±0.40 | - |
| **2** | 6.70±0.03 | 5.56±0.43* | 3.34±0.15 | 3.29±0.38 | 11.86±1.66 | 12.06±0.78 |
| **4** | 6.73±0.60 | 6.06±0.52 | 2.75±0.25 | 2.50±0.65 | 11.12±0.68 | 11.46±0.36 |
| **6** | 6.00±0.57 | 5.88±0.47 | 2.67±0.15 | 2.47±0.24 | 11.76±0.90 | 11.32±0.67 |
| **8** | 4.17±0.16 | 3.76±0.25* | 2.47±0.18 | 1.96±0.31* | 8.82±0.33 | 7.93±0.52* |

### Example 2

The following is an example of a filled gelatin capsule according to the invention. A composition comprising pinolenic acid is encapsulated into a gelatin capsule according to methods well-known in the art. The resulting encapsulated product contains 500 mg of pinolenic acid and one capsule can be taken up to four times daily by an adult human.

Soft gel capsules are produced by rotary die processing. The material for the outside shell of the capsules, the gel, and the fill are formulated separately. Once the gel mass and the fill mass are ready, the gel is spread into thin film to form two gelatin ribbons which are then rolled over two separate dies which determine the size and the shape of the capsules. As the gelatin films adapt to the dies, the fill is carefully dosed to a level of 500 mg, 750 mg or 1000 mg oil per capsule and injected between the two gelatin ribbons which are sealed immediately afterwards by applying heat and pressure. Capsules fall from the machine and are then dried under a stream of hot air.

## Claims

1. Use of pinolenic acid or a derivative thereof in the manufacture of a composition for treating or preventing a condition of the liver.

2. Use as claimed in Claim 1, wherein the pinolenic acid or derivative represents at least 75 % by weight of the total Δ5-polyunsaturated C18-C20 fatty acids in the composition.

3. Use according to Claim 1 or Claim 2, wherein the condition is fatty liver.

4. Use according to any one of Claims 1 to 3, wherein the composition is in the form of a food supplement, a pharmaceutical product or a food product.

5. Use according to any one of Claims 1 to 4, wherein the composition comprises pinolenic acid or a derivative thereof in a form selected from free acid, salt, mono-, di- or triglyceride, or mixtures thereof.

6. Use according to any one of Claims 1 to 5, wherein the composition is pine nut oil or is derived from pine nut oil.

7. Use according to Claim 6, wherein the pine nut oil is from *Pinus koraiensis.*

8. Use according to any one of the preceding claims, wherein the composition additionally comprises a fatty acid or derivative thereof selected from the group consisting of linoleic acid, oleic acid, conjugated linoleic acid, enriched isomer mixtures of conjugated linoleic acid, EPA and DHA, and mixtures thereof.

9. Use according to Claim 8 wherein the fatty acid or derivative thereof is present as a free fatty acid, salt, mono-, di- or triglyceride or a mixture thereof.

10. Use according to any one of the preceding claims, wherein the composition comprises from 1 to 50 % by weight pinolenic acid or derivative thereof, based on the total weight of fatty acids in the composition (calculated as free fatty acid).

11. Use according to any one of the preceding claims, wherein the composition comprises from 30 to 70 % by weight linoleic acid or derivative thereof, based on the total weight of fatty acids in the composition (calculated as free fatty acid).

12. Use according to any one of the preceding claims, wherein the composition comprises from 10 to 40 % by weight oleic acid or derivative thereof, based on the total weight of fatty acids in the composition (calculated as free fatty acid).

13. Use according to any one of the preceding claims, wherein the composition comprises from 1 to 15 % by weight palmitic acid or derivative thereof, based on the total weight of fatty acids in the composition (calculated as free fatty acid).

14. Use according to Claim 1, wherein the composition is a food product.

15. Use according to Claim 14, wherein the food product comprises a glyceride selected from: liquid oils, including soybean oil, sunflower oil, rape seed oil and cotton seed oil; cocoa butter and cocoa butter equivalents; palm oil and fractions thereof; enzymically made fats; fish oils and fractions thereof; conjugated linoleic acid and enriched isomer mixtures thereof; gamma linoleic acid and enriched mixtures thereof; hardened liquid oils; and mixtures thereof.

16. Use according to Claim 14 or Claim 15, wherein the composition is a food product selected from the group consisting of: margarines; low fat spreads; very low fat spreads; bicontinuous spreads; water continuous spreads; confectionary products, such as chocolates, coatings or fillings; ice creams; ice cream coatings; ice cream inclusions; dressings; mayonnaises; sauces; bakery fats; shortenings or cheese; meal replacement products; health bars; muesli bars; drinks; dairy products; low carbohydrate products; low calorie products; soups; cereals and milk shakes.

17. Use according to any one of Claims 1 to 13, wherein the composition is a pharmaceutical product in a form selected from the group consisting of tablets, pills, capsules, caplets, multiparticulates including: granules, beads, pellets and micro-encapsulated particles; powders, elixirs, syrups, suspensions and solutions.

18. Use according to any one of Claims 1 to 13, wherein the composition is a food supplement in the form of a soft gel or a hard capsule comprising an encapsulating material.

19. Use according to Claim 18, wherein the encapsulating material is selected from the group consisting of gelatin, starch, modified starch, and starch derivatives.

20. Use according to any one of Claims 1 to 19 wherein the composition lowers liver cholesterol, liver triglycerides and liver phospholipids.

21. Use according to any one of Claims 1 to 20, wherein the liver weight is decreased by at least 20%.

22. A method of treating or preventing a liver condition, which comprises administering to a mammal an effective amount of pinolenic acid or a derivative thereof.

23. Composition comprising pinolenic acid or a derivative thereof for use in treating or preventing a condition of the liver.

24. Method as claimed in Claim 22 or composition as claimed in Claim 23, having the features described in any one of Claims 2 to 21.
